# EUROPEAN PATENT APPLICATION

(11) **EP 3 936 157 A1**
(43) Date of publication of application: **12.01.2022**
(21) Application number: 21184602.7
(22) Date of filing: 08.07.2021
(51) Int. Cl.: A61L 2/08, A61L 2/10

(54) **SELF-CLEANING DEVICES USING LIGHT SOURCES ARRANGED NEAR THE DEVICE SURFACE**

(30) Priority: 10.07.2020 US 202063050177 P; 07.07.2021 US 202117369423
(71) Applicant: Accenture Global Solutions Limited, Dublin 4 (IE)
(72) Inventor: MAHESHWARI, Aditi, San Francisco, CA 94105 (US); GREENSPAN, Mark Benjamin, Oakland, CA 94618 (US); DANIELESCU, Lavinia Andreea, San Francisco, CA 94110 (US)
(74) Representative: Swindell & Pearson Limited

(57) **Abstract**

This document describes self-cleaning devices. In one aspect, a self-cleaning device includes a rigid or semi-rigid surface covered with a photocatalyst, one or more light sources disposed under or above the surface, and a triggering mechanism that activates a cleaning cycle by activating the one or more light sources.

## Description

### CROSS-REFERENCE TO RELATED APPLICATIONS

This application claims the benefit of U.S. Patent Application No. 63/050,177, filed July 10, 2020, which is incorporated herein by reference.

### FIELD

This specification generally relates to self-cleaning devices.

### BACKGROUND

Many surfaces are contacted by multiple people throughout the day. These surfaces are typically cleaned manually on a periodic basis. However, such periodic cleaning may not be sufficient to prevent the spread of organic contaminants and germs prior to the next person contacting the surface.

### SUMMARY

This specification generally describes self-cleaning devices that include a rigid or semi-rigid surface that has a coating that is activated for cleaning the surface(s) of the devices using visible, ultraviolet (UV), e.g., far-UVC, or UV-Visible light. The coating can be a photocatalyst that is activated by a light source and that, when activated, cleans and/or decontaminates the surface. When the light source is on, the light emitted by the light source activates the photocatalyst, causing the photocatalyst to clean and/or decontaminate the surface of the device. The incidence of the light initiates a photocatalytic reaction, whereby carbon-based organic compounds on the surface, such as bacteria, odors, grease, oils, etc., are chemically broken down into simpler molecules that are then dispersed away from the surface.

In some implementations, the light source can be disposed under the surface that includes the photocatalyst coating. The surface can be transparent or translucent enabling the light emitted by the light source to illuminate the surface and activate the photocatalyst. In another example, the surface can include holes that enable the light to illuminate the surface. The light source can include light emitting diodes (LEDs) or optical fibers disposed under the surface.

In some implementations, the light source can be disposed above the surface. For example, the light source can include one or more LEDs, one or more incandescent light bulbs, or other visible lamp(s).

According to some implementations, a self-cleaning device includes a rigid or semi-rigid surface covered with a photocatalyst, one or more light sources disposed under or above the surface, and a triggering mechanism that activates a cleaning cycle by activating the one or more light sources.

Implementations may include one or more of the following features. In some implementations, the one or more light sources can include one or more light emitting diodes that emit light within a particular light spectrum, e.g., the visible spectrum, the UV light spectrum, or a UV-Visible light spectrum that includes at least a portion of the UV light spectrum and the visible light spectrum.

The one or more light sources can be arranged facing an underside of the rigid or semi-rigid surface. The one or more light sources can be arranged facing a direction that extends substantially in parallel to the rigid or semi-rigid surface.

The photocatalyst can include titanium dioxide or zinc oxide. The photocatalyst can include titanium dioxide or zinc oxide doped with one or more elements. The one or more elements can include one or more of lithium, sodium, magnesium, iron, cobalt, chromium, gold, vanadium, manganese, carbon, boron, iodine, fluorine, sulfur, nitrogen or rare earth elements.

The surface can include one or more holes that allow light emitted by the one or more light sources to illuminate the surface. The self-cleaning device can include a transparent or translucent layer arranged above the surface that includes one or more holes that allow light emitted by the one or more light sources to illuminate the surface. The surface can be transparent or translucent.

The triggering mechanism can include a touch sensor. The triggering mechanism can be configured to activate the cleaning cycle in response to detecting that the surface has been touched. The triggering mechanism can include a pressure sensor. The triggering mechanism can be configured to activate the cleaning cycle in response to detecting an increase in pressure applied to the surface.

The surface can include one or more regions that each include a corresponding light source that is independently activated to clean a corresponding surface of the region.

The self-cleaning device can be in the form of a handrail or a flat horizontal surface in some instances. The self-cleaning device can include one or more buttons for receiving touch input. The surface can include an outer surface of the one or more buttons.

According to another implementation, a method for manufacturing a self-cleaning device includes obtaining a rigid or semi-rigid surface, coating the surface with a photocatalyst that is activated by light within a particular light spectrum (e.g., the visible spectrum, the ultraviolet spectrum, or a UV-Visible light spectrum that includes at least a portion of the ultraviolet (UV) light spectrum and the visible light spectrum) and embedding one or more light sources under the surface.

In some instances, obtaining the surface can include creating a pattern of holes that extend through the surface. The method can include arranging a transparent or translucent refractive layer above the pattern of holes. Coating the surface with a photocatalyst can include abrading the surface. Coating the surface with a photocatalyst can include applying a primer or adhesive to the surface.

The methods in accordance with the present disclosure can include any combination of the aspects and features described herein. That is, methods in accordance with the present disclosure are not limited to the combinations of aspects and features specifically described herein, but also may include any combination of the aspects and features provided.

The subject matter described in this specification can be implemented in particular embodiments so as to realize one or more of the following advantages.

In accordance with an aspect of the present disclosure, a surface of a self-cleaning device is covered, e.g., fully or partially, with a photocatalyst. When the photocatalyst is activated by light, reactive substances, e.g., hydroxyl radicals and superoxide radical anions, are formed. These reactive substances decompose organic compounds (e.g., to clean stains), eliminate bad smells, and kill organic contaminants, germs, bacteria, and/or other pathogens. Titanium dioxide in different types and forms have shown great potential as a powerful photocatalyst for various significant reactions due to its chemical stability, nontoxicity, and high reactivity. By leveraging these and other photocatalyst coatings, in addition to hydrophobic or polytetrafluoroethylene nanoparticles coatings, surfaces can be created that are easy to clean infrequently, that can repel liquids and bodily fluids, and that can self-clean during the day reducing the potential for viruses and bacteria to build up on surfaces in public spaces and infect individuals.

This cleaning cycle can be initiated by various triggering mechanisms, initiated periodically, or can always be on to continuously clean the surface. For example, the self-cleaning device can include triggering mechanism in the form of a touch sensor and a controller that initiates a cleaning cycle in response to detecting that the surface has been touched. By embedding the light source(s) under the surface and using touch sensors (or other triggering mechanism), the self-cleaning process is automatically initiated as soon as the surface is dirtied, irrespective of the presence of ambient light. For example, the cleaning cycles can be initiated and completed in dimly lit and indoor areas in which UV light may not typically reach the surface to activate the photocatalyst. The cleaning cycles can be initiated and completed in outdoor areas to accelerate the cleaning process triggered by UV light, e.g., by sunlight. The use of light sources embedded under the surface make the self-cleaning devices described in this document particularly advantageous for such indoor surfaces. The triggering mechanism also ensures that the cleaning process is initiated only when needed, thereby conserving energy that would otherwise be consumed by the light sources that are always on or that are triggered periodically even when the surface has not been touched or otherwise soiled. Not only does the use of photocatalyst coatings help clean the surface, they can also convert harmless gases such as nitrogen oxide (NOx) in the atmosphere to harmless components reducing air pollution, e.g., in crowded areas.

The details of one or more implementations of the present disclosure are set forth in the accompanying drawings and the description below. Other features and advantages of the present disclosure will be apparent from the description and drawings, and from the claims.

### BRIEF DESCRIPTION OF THE DRAWINGS

FIGS. 1A-1C depict an example self-cleaning device.
FIG. 2 is a schematic overview of an example cleaning cycle for self-cleaning devices.
FIG. 3 depicts example self-cleaning devices.
FIGS. 4A-4C depict a further example of a cleaning cycle for a self-cleaning device.
FIGS. 5-7 depict further examples of self-cleaning devices.
FIG. 8 is a schematic overview of an example method of manufacturing a self-cleaning device.

### DETAILED DESCRIPTION

This document generally describes self-cleaning devices and methods for manufacturing self-cleaning devices. The self-cleaning devices can include one or more surfaces, e.g., one or more rigid or semi-rigid surfaces, coated with a photocatalyst. The self-cleaning devices can also include, or be placed in an area that includes, a light source that illuminates the surface(s) to activate the photocatalyst and initiate a photocatalytic reaction that cleans and decontaminates the surface.

The self-cleaning devices can be used for high-touch surfaces, such as railings (e.g., handrails), automated teller machines (ATMs), vending machines, point of sale (POS) terminals, kiosks, mass transit handles and poles (e.g., subway handles), surfaces in theaters, stadiums, and amusement parks (e.g., railings, poles, door handles, etc.), and/or other surfaces that are commonly touched by different people. For example, as described below, a self-cleaning handrail can include a transparent surface or a surface with holes with a light source disposed under the surface to illuminate the surface and activate the photocatalyst. By including a light source under the surface or an external light source that is arranged to illuminate the surface, the light source can be used to clean indoor surfaces or other surfaces that are not exposed to natural sunlight.

The self-cleaning devices can include a triggering mechanism that initiates cleaning cycles at appropriate times. For example, the triggering mechanism can include sensors that can detect when the surface, or a region of the surface, has been touched. In response, the triggering mechanism can initiate a cleaning cycle by turning on the light source. In some implementations, the light source may always be on to continuously clean the surface. For example, a ceiling light in an indoor area may always be on, or on for lengthy continuous time periods (e.g., during the work day), to clean surfaces in that area.

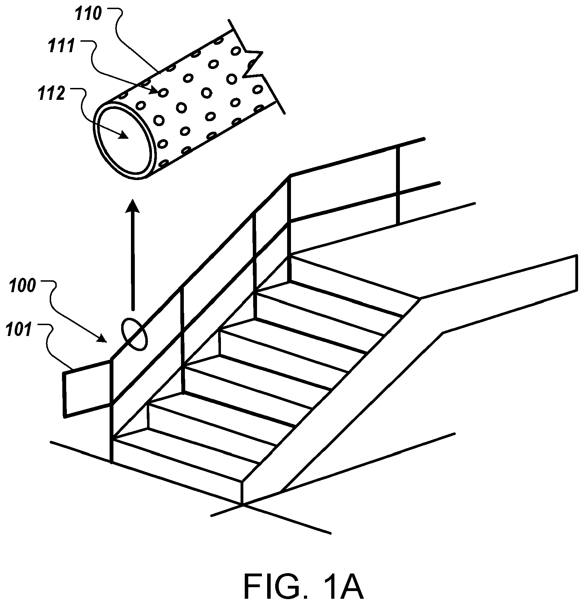

FIG. 1A depicts an example self-cleaning device 100. In this example, the self-cleaning device 100 is in the form of a handrail 101 with a self-cleaning external surface 110. The surface 110 can be coated with a photocatalyst that is activated by visible light and/or ultraviolet (UV) light. For example, the surface 110 can be coated with a photocatalyst that is activated by light within a UV-Visible light spectrum that includes at least a portion of the UV spectrum and the visible spectrum that is visible to the human eye. In another example, the surface 110 can be coated with a photocatalyst that is activated by visible light or a coating that is activated by UV light. The UV light may be of the UVC wavelength (100 ― 280 nm), specifically UVGI (254 nm), far-UVC (222 nm) or any other UV wavelength. As described below, the surface 110 can be coated by a photocatalyst that is activated by far-UVC light to still kill pathogens while still being safe for humans.

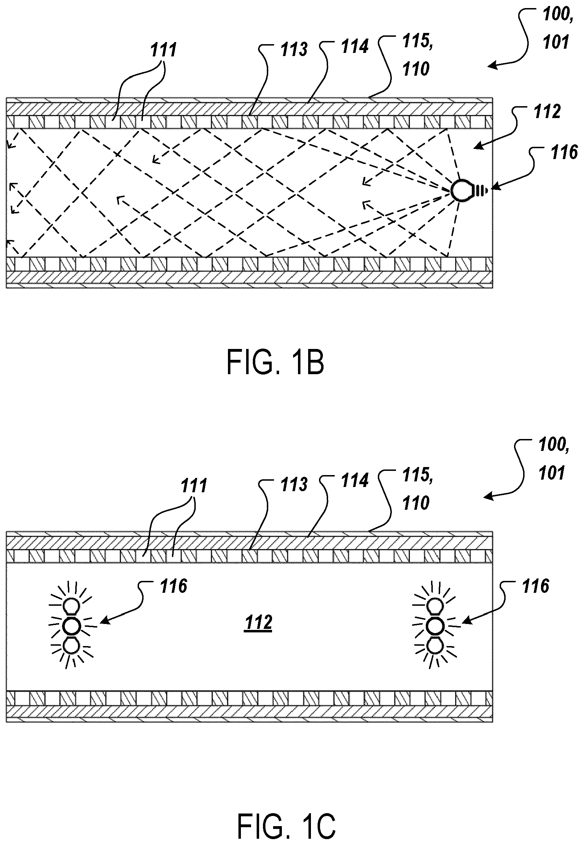

The handrail 101 includes multiple holes 111 arranged along the surface. The handrail 101 can also include a light source disposed within an interior 112 of the handrail 101, as shown in FIGS. 1B and 1C. The light source can include one or more LEDs (or other appropriate light) disposed within the interior 112 of the handrail 101 and arranged to emit light through the holes 111 to illuminate the surface 110. The holes 111 can be configured to ensure that the entire external surface 110, or at least a threshold amount (e.g., 90%, 95%, etc.) of the handrail 101 is illuminated and therefore cleaned by the activated photocatalyst.

For example, the pattern of the holes 111, the distance between adjacent holes 111, and/or the angle of the holes 111 can be configured such that the light source illuminates the entire surface 110. The holes 111 are shown as circles but can be created from any shape. In some instances, the holes 111 extend perpendicular to the length of the handrail 101 to allow for maximum light exposure from the light source. For example, the holes 111 can be arranged in a consistent recurring circular pattern that results in equal distance between all the holes. However, the holes 111 can be arranged in an artistic or geometric pattern that additionally provides a self-cleaning effect. For a handrail 101 having a diameter between 0.1 and 5 inches, the holes 111 can have a diameter that ranges from about 0.001 to 0.5 inches. Other appropriate dimensions and relative dimensions can also be used.

FIGS. 1B and 1C depict partial cross-sectional views of example self-cleaning devices 100. As in FIG. 1A, the self-cleaning device 100 may be a handrail 101. The self-cleaning device 100 can include an elongate tube 113 with the holes 111 extending through a sidewall of the tube 113. For example, the tube 113 can be made of metal or stiff plastic to increase the structural integrity of the handrail 101. Other rigid or semi-rigid materials can also be used.

One or more light sources 116 are arranged in the interior 112 of the tube 113. For example, the light sources 116 may include one or more LED lights and a mount (not shown) that secures the light sources 116 to the tube 113. For example, a LED light can be housed in a cone-shaped mount that is held in place in the interior 112 of the tube 113 using a web-shaped or lattice-shaped holder. The web-shaped holder can be attached to the interior walls of the tube 113, e.g., using adhesives, to keep the LED light in place.

As shown in FIG. 1B, the light source 116 may be arranged to emit light along a longitudinal axis of the tube 113. In this example, there may be a single light source 116 within the tube 113 or multiple light sources 116 within the tube 113, e.g., one located at each end of the tube 113. As shown in FIG. 1C, multiple light sources 116 can be arranged at intervals along the longitudinal axis of the tube 113 and configured to emit light substantially perpendicular to the axis. In some instances, the light sources 116 may align with a hole 111 in the tube 113, e.g., with a light source 116 for each hole 111. Alternatively, the light sources 116 may be offset from the holes 111 to allow light to reflect inside of the tube 113. For example, the interior walls of the tube 113 can be made of, or coated with, a reflective material that reflects light within the tube 113 to enable the light to reach holes 111 arranged away from the light sources 116.

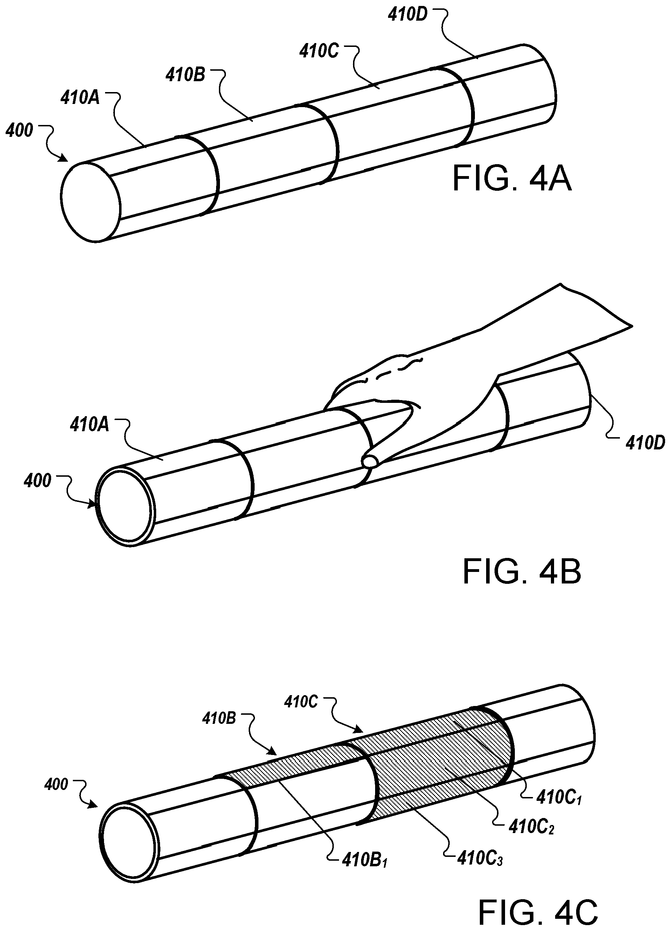

As also described below with reference to FIGS. 4A-4C, a self-cleaning device can include multiple regions that are cleaned independently of each other. In such examples, each region can have a corresponding light source that is activated during a cleaning cycle for the region. In FIG. 1C, each region can include a light source 116 disposed under the region and that shines through the holes 111 of that region. The light sources 116 can be activated independently of each other. In some implementations, individual LEDs within a single light sources 116 can be activated independently from the other LEDs in the light source 116.

For region-specific activation, a controller can provide a respective output to each light source 116, e.g., via a wired or wireless connection. To activate the light source 116 for a particular region, e.g., in response to detecting that the region has been touched or otherwise potentially soiled, the controller can activate the light source 116 for that region via the wired or wireless connection. For example, the controller can activate a light source 116 by applying a voltage across the light source 116.

In some implementations, an external light source (not shown) can be used to activate the photocatalyst on the surface 110. In such implementations, the surface 110 may not include the holes 111. Instead, an external light source (e.g., visible, UV, or UV-Visible) may be activated to illuminate the surface 110 of the handrail during each cleaning cycle.

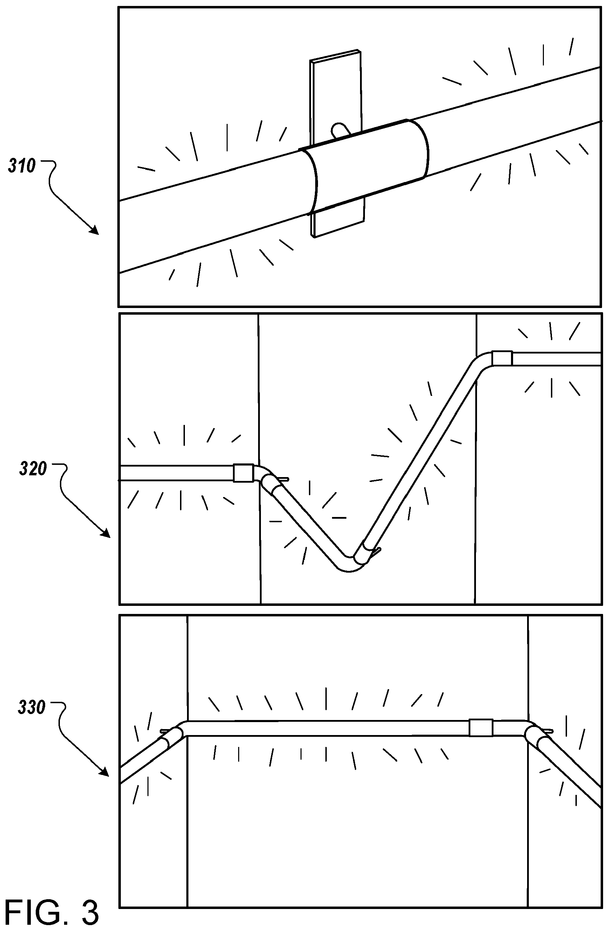

In some implementations, a transparent or translucent refractive layer 114 is layered on an outer surface of the tube 113. The layer 114 may diffuse the light emitted through the holes 111 in the tube 113 along the surface 110 of the handrail. In some instances, the layer 114 may be formed of plastic, e.g., acrylic or polycarbonate plastics. The thickness of the layer 114 may range from about 0.01 to 0.2 inches (0.3 to 5.1 mm), e.g., 0.01 to 0.2 inches (0.3 to 5.1 mm) for acrylic material, 0.06 to 0.12 inches (1.5 to 3.0 mm) for a polycarbonate sheet, or 0.01 to 0.02 inches (0.3 to 0.5 mm) for a polycarbonate film. As shown in FIG. 3, some implementations may include a transparent or translucent refractive layer without an inner tube 113. In such cases, the thickness of the refractive layer may be greater than the thickness of the layer 114 in FIGS. 1B and 1C.

An outer surface of the layer 114 can be coated with a photocatalyst 115, such as a type or form of titanium dioxide (TiO2) or zinc oxide (ZnO). The photocatalyst coats the outer surface 110 and, thus, the surface of the handrail 101 that is touched. When the photocatalyst is activated by light, reactive substances, e.g., hydroxyl radicals and superoxide radical anions, are formed. These reactive substances decompose organic compounds (e.g., to clean stains), eliminate bad smells, and kill organic contaminants, germs, and/or bacteria. In the implementation depicted in FIG. 1A, the handrail 101 does not include a refractive layer 114. As such, the photocatalyst is applied directly to the surface of a metal tube.

Visible light-responsive photocatalysts can be created by adding small amounts of cations and metal oxides by both chemical doping and physical ion-implantation methods to normally purely UV-active TiO2. Other modification methods include impurity doping (chemical and physical), semiconductor coupling, dye sensitization, etc. Traditional photocatalysts respond to ultraviolet light that can be produced in a variety of wavelengths (e.g., 100-400 nanometers (nm)) but some of these (far-UVC excluded) can cause damage to human tissues such as eyes and skin. Instead of using potentially harmful wideband UV light, a specific wavelength (e.g. 222 nm) within far-UVC may be chosen instead. The TiO2 coating can be modified by doping with abovementioned elements to have antibacterial and cleaning effects when activated by visible light (400-700 nm) alone, as described above.

The following materials are effective in enhancing TiO₂'s photocatalytic properties with visible light: doping of TiO2 nanoparticles with Li, Na, Mg, Fe and Co nitrates; deposition of Au onto TiO2; doping of TiO2 with transition metals such as Cr, Fe and V; doping with rare earth elements; and doping of TiO₂ with non-metal dopants such as C, B, I, F, S and N.

For self-cleaning purposes, some (non-exhaustive) modifications to TiO₂ include TiO₂/SiO₂/graphene oxide nanocomposites; porphyrin dye/TiO₂ coating used for PET fibers; N-TiO₂ film; manganese doped TiO₂ nanoparticles; TiO₂ films modified with Au nanoclusters; TiO₂-Al₂O₃ coatings; and TiO₂/Pt/WO₃ hybrid films. Zinc oxide and be modified in similar ways to provide a visible light-responsive photocatalyst.

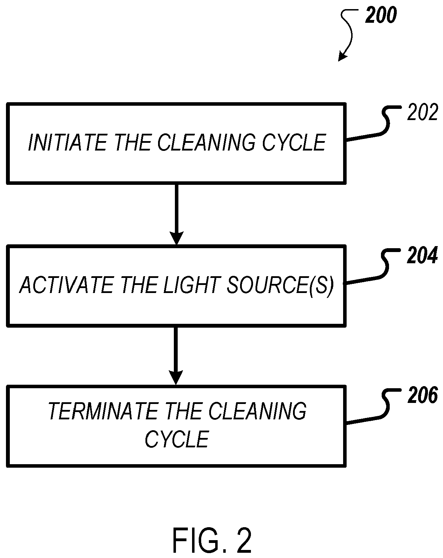

FIG. 2 is a schematic overview of an example cleaning cycle 200. The cleaning cycle 200 can include initiating 202 the cleaning cycle, activating 204 one or more onboard and/or external light sources, and terminating 206 the cleaning cycle. The cleaning cycle 200 is applicable to any of the self-cleaning devices of the present disclosure. For brevity, the cleaning cycle is described with reference to the handrail 101 of FIG. 1.

The cleaning cycle can be initiated using a triggering mechanism that initiates the cleaning cycle, e.g., activates the light source(s) to illuminate the surface. In some implementations, the handrail 101 includes a controller, e.g., a microcontroller, and a touch sensor for detecting when the handrail 101 is touched. In this example, the controller can activate the light source(s) in response to the touch sensor detecting that the handrail 101 has been touched. The controller can activate the light source(s) immediately, after a brief delay period (e.g., 1-3 seconds), or after detecting that the handrail 101 is no longer being touched.

In implementations in which the external surface 110 of the handrail 101 is not rigid, e.g., semi-rigid such that the surface can be pressed inwards at least a small amount, the touch sensor can be include a pressure-sensitive sheet, e.g., Velostat^{™}, force sensitive resistors, or another appropriate type of pressure sensor disposed under the surface 110 of the handrail 101. For example, in FIG. 1B and 1C, the pressure sensor (not shown) can be disposed between the refractive layer 114 and the tube 113 and include holes that are aligned with the holes 111 in the tube 113. In implementations in which the outer surface is rigid, other touch sensors can be used such as capacitive touch sensors or heat sensors. For example, a heat sensor can be used to detect changes in temperature consistent with human touch, e.g., at least a threshold increase in temperature over a short period of time such as 1-3 seconds or another appropriate time period.

If a touch is detected, the controller can initiate the cleaning cycle depicted in FIG. 2. The cleaning cycle includes activating the light source(s) for a period of time, thereby initiating the photocatalyst reaction. The period of time can be a specified period of time for each cleaning cycle. This specified period of time can be set for the handrail 101 or a particular region of the handrail 101 can be based on the expected duration needed to clean the surface 110, e.g., based on the amount of traffic or the number of people expected to come into contact with the handrail 101 or the region of the handrail 101.

The example cleaning cycle 200 includes terminating 206 the cleaning cycle 200 by deactivating, i.e., turning off, the one or more light sources. In some instances, the light sources are deactivated in response to a timer for the cleaning cycle expiring. In instances in which the cleaning cycle 200 is initiated manually, the cleaning cycle 200 can also be terminated manually.

FIG. 3 depicts example self-cleaning devices 310, 320, and 330. These self-cleaning devices 310-330 are in the form of handrails with a transparent or translucent surface having a photocatalyst, e.g., one of the photocatalysts described above, coated thereon. The handrails can also include one or more light sources disposed within the handrails in a similar manner to the light sources depicted in FIG. 1B and 1C. The light sources can emit visible, UV, or UV-Visible light through the transparent or translucent surface to illuminate the outer surface and activate a photocatalyst coated on the outer surface during each cleaning cycle. The self-cleaning devices 310-330 can also include a controller and sensor for activating the cleaning cycles, similar to the self-cleaning device 100 of FIGS. 1A-1C. However, in this example, the outer surfaces of the handrails are illuminated via the transparent or translucent surfaces rather than through holes.

FIG. 4A-4C depict a cleaning cycle for a self-cleaning device 400. As in FIGS. 1A-1C and FIG. 3, the self-cleaning device 400 may be a handrail. The self-cleaning device 400 includes multiple self-cleaning regions, including sections 410A-410D. Each region can include a corresponding touch sensor for detecting when the region is touched. Each region can also include a corresponding light source that is configured to illuminate the surface of the region during a cleaning cycle for the region. The light source for a region can be disposed under the region (e.g., if the region is transparent, translucent, or includes holes) or external to the region but directed at the region.

Referring again to FIG. 2, the cleaning cycle for each region can be initiated individually. As shown in FIG. 4B, a person has touched the external surface of several regions of the self-cleaning device 400, but not all of the regions. For example, the user's hand does not touch the regions 410A and 410D of the self-cleaning device 400. In response, a cleaning cycle has been initiated for regions touched by the user, namely 410B and 410C.

In some instances, the regions 410B and 410C can be split into even smaller sub-regions 410B₁, 410Ci, 410C₂, 410C₃. For example, the regions may correspond to a respective light source 116 depicted in FIG. 1C, while the sub-regions correspond to an individual LED of a particular light source 116. As shown in FIG. 4C, the cleaning cycle can be initiated on a subregion basis for sub-regions that were touched by the user. Although the regions and sub-regions in FIGS. 4A-4C are depicted as distinct regions, in some implementations, the regions and/or sub-regions may overlap with one another.

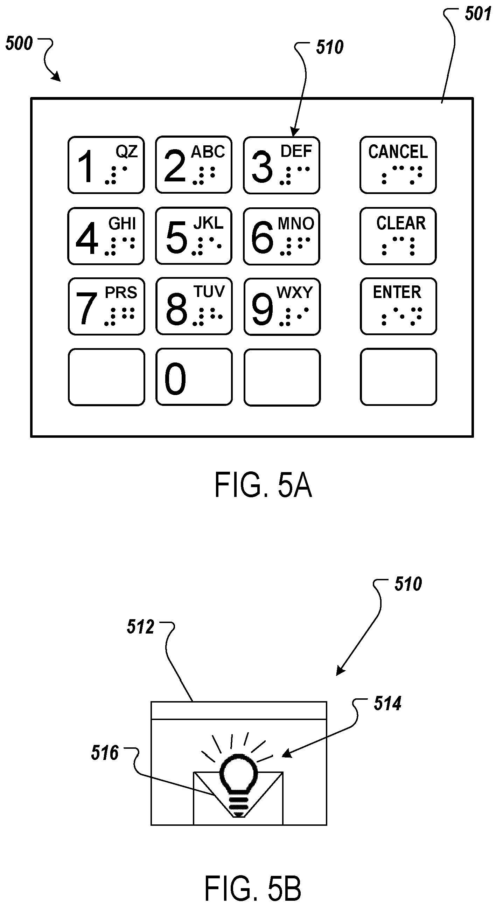

FIGS. 5A and 5B depict a further example of a self-cleaning device 500. In this example, the self-cleaning device 500 is in the form of a keypad 501, e.g., for an ATM. The self-cleaning device 500 can be used for other types of keypads, such as checkout terminals, arcade games, ticketing systems, etc. The buttons 510 of the keypad 501 can be coated with a photocatalyst, e.g., one of the photocatalysts described above. The self-cleaning device 500 can include one or more light sources that illuminate the surface of the buttons 510 during cleaning cycles, as shown in FIG. 5B.

In some implementations, at least a portion of a surface 512 of the buttons 510 is transparent or translucent and coated with a photocatalyst. In this example, the self-cleaning device 500 can include a UV-Visible light or far UVC light (e.g., LED) under the surface 512 of each key 510 to illuminate the outer surface 512 of the buttons 510. For example, a LED 514 may be mounted in a reflective cone 516 and configured to emit light toward an underside of the button surface 512. The transparent or translucent material of the surface 512 may transmit light to activate the photocatalyst coating the surface 512. In other implementations, one or more light sources may be provided for the entire keypad 501, as opposed to individual buttons 510. Instead of a light source under the surface 512, the self-cleaning device 500 can include one or more light sources that are arranged to shine light onto the outer surface 512 of the buttons 510.

The self-cleaning device 500 can initiate a cleaning cycle in response to detecting that one or more of the buttons 510 have been touched. For example, a cleaning controller of the self-cleaning device 500 can be integrated with the hardware and/or software of the device that detects when the buttons 510 are pressed. In a particular example, if the self-cleaning device 500 is an ATM, the cleaning controller can be integrated with the ATM's hardware of software for detecting when the buttons 510 are pressed to receive data indicating when each button 510 is pressed. For example, if a person presses the button for number 1 as part of entering a personal identification number (PIN), the ATM can detect that the number 1 button was pressed in its normal manner. The controller can be integrated with the ATM to receive data indicated that this button was pressed and initiate a cleaning cycle for the button in response to receiving the data.

When a button 510 is pressed, the cleaning controller can initiate a cleaning cycle for the button 510 that was pressed or for all buttons 510. As shown in FIG. 5B, each button 510 can include a corresponding light source 514 that the cleaning controller activates when or after the button 510 is pressed. In some implementations, the controller can be configured to initiate the cleaning cycle after the person has finished using the ATM or other device that has the buttons 510. For example, the controller can initiate the cleaning cycle after a specified period of time has elapsed since any button was last pressed. In another example, if the controller is integrated with the ATM or other device, the controller can detect that a user session has ended.

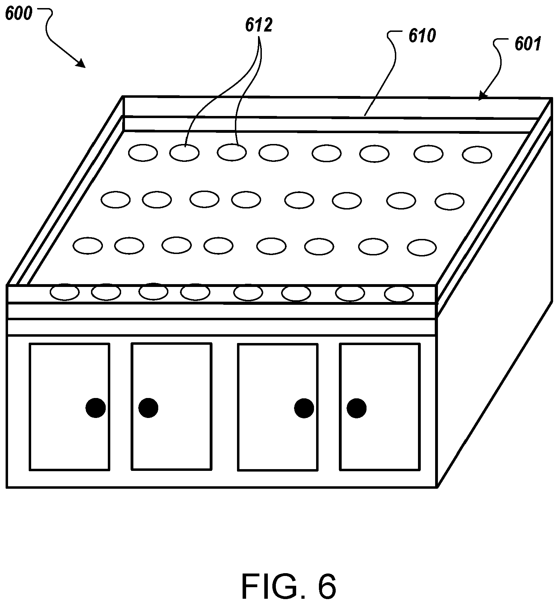

FIG. 6 depicts a further example of a self-cleaning device 600. In this example, the self-cleaning device 600 is in the form of a countertop 601. The countertop 601 includes a work surface 610 made of transparent or translucent material. An outer surface of the work surface 610 is coated with photocatalyst. Light sources 612 (e.g., individual LEDs) can be arranged below the work surface 610. The light sources 612 are configured to transmit light through the material of the work surface 610 and activate the photocatalytic coating on the work surface 610.

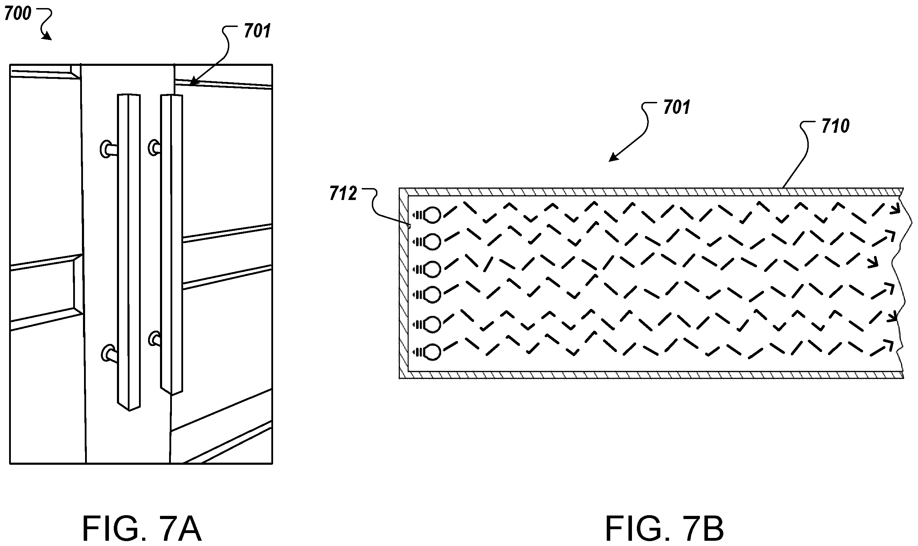

FIGS. 7A and 7B depict a further example of a self-cleaning device 700. In this example, the self-cleaning device 700 is in the form of a door handle 701. The door handle 701 includes an outer surface 710 made of transparent or translucent material and coated with a photocatalyst. As shown in the partial cross-section of FIG. 7B, light sources 712 are arranged at one end of the door handle 701 and configured to emit light along a length of the door handle 701. The light from the light sources 712 is refracted through the transparent or translucent material of the outer surface 710 to activate the photocatalyst. In this sense, the door handle 701 is similar to the self-cleaning devices 310, 320, 330 shown in FIG. 3.

In some implementations, the door handle 701 may include an inner layer that comprises a perforated metal, similar to the examples of FIGS. 1B and 1C. In other implementations, the door handle 701 may include light sources 712 that are oriented towards, i.e., substantially perpendicular to the outer surface 710.

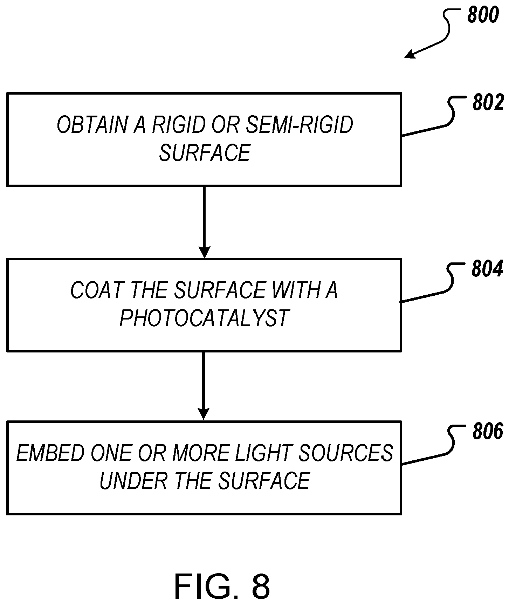

FIG. 8 is a schematic overview of an example method 800 of manufacturing a self-cleaning device. For example, the method 800 can be used to manufacture any of the self-cleaning devices described above. The method 800 includes obtaining 802 a rigid or semi-rigid surface that forms a touch surface, i.e., outer surface, of the device, coating 804 the surface with a photocatalyst, and embedding 806 one or more light sources under the surface.

The surface may be rigid, i.e., cannot be pressed inwards at least a small amount, or semi-rigid, i.e., can be pressed inwards at least a small amount. A semi-rigid surface may be a surface that is stiff and solid, but not inflexible. For example, a semi-rigid surface may be capable of bending or being deformed a small amount, or may have at least a threshold stiffness measure (e.g., in pounds per inch). For instance, metal surfaces may be rigid or semi-rigid, depending on their thickness and the presence of perforations or holes. Plastic surfaces, such as those described above, may be rigid or semi-rigid depending on their thickness.

In some instances, obtaining 802 the surface can include creating a pattern of holes or perforations that extend through the surface. The method 800 may optionally include arranging a transparent or translucent refractive layer above the perforations to form the touch surface.

In some instances, coating 804 the surface with a photocatalyst can include pre-treating the surface. Pre-treating the surface can include abrading the surface or coating the surface with a primer or adhesive to name a few examples.

In addition to or separately from a pre-treatment process, coating 804 the surface with a photocatalyst can utilize spraying, painting, direct coating or floating knife coating, direct roll coating, or padding techniques to name a few examples. In instances in which a pre-treatment process has been performed, the coating may be applied as soon as possible after the pre-treatment process. In some instances, a flexible sheet can be pre-treated and/or coated and then placed on top of or attached to an underlying structure to form a coated rigid or semi-rigid surface. For example, a pre-treated and coated plastic laminate may be attached to the top surface of a table or countertop, as shown in FIG. 6.

Direct coating or knife coating applies a viscous photocatalyst to the surface while the surface is run below a knife blade. The distance between the surface and the knife blade can be adjusted to adjust the thickness of the coating. The angle between the surface and the knife blade can also be adjusted to modify coverage of the coating on the surface.

Direct roll coating uses a roller suspended in a liquid photocatalyst solution to roll the solution across the surface. Excess solution may be scraped from the roller using a blade arranged adjacent to the roller.

Padding can include submerging the surface in a liquid photocatalyst solution and using rollers to remove the excess solution.

In the present examples, coating 804 the surface with a photocatalyst can further include drying, and optionally curing, the coated surface.

In some instances, the method 800 further includes coating the surface with a hydrophobic coating before or after the surface has been coated with the photocatalyst. The hydrophobic coating or super-hydrophobic coating may be configured to repel water or other liquids. For example, if a liquid is spilled onto the self-cleaning device, the hydrophobic coating may cause the liquid to form beads that roll off the surface of the device. Any residual contaminants may be neutralized using the process described in reference to FIG. 2.

The method 800 further includes embedding 806 one or more light sources under the surface. Example arrangements are shown in FIG. 1B, 1C, 5B, 6, and 7B. In some instances, the one or more light sources can include LEDs. The LEDs can be visible light LEDs that emit visible light, ultraviolet (UV) lights that emit UV light, depending on the photocatalyst material. The light sources can include LED strands, LED fibers, fiber optics, or electroluminescent wires. The light sources can include individual LEDs that are attached to an underside of the outer surface. The light sources can also include printed LEDs. The number of light sources may vary. For example, as shown in FIG. 1B, a single large light source 116 may be arranged to illuminate the outer surface 110. Such a light source may emit fluorescent or incandescent light.

The light sources can be arranged in a pattern such that the light sources emit light that diffuses and hits every part of the coated surface. For example, the one or more light sources can be arranged facing an underside of the outer surface. In other examples, the one or more light sources can be arranged in a direction parallel to the outer surface. Independently of the direction in which the one or more light sources are facing, the method 800 may further include providing a reflective or refractive layer adjacent to the one or more light sources, such as the reflective cone shown in FIG. 5B. For example, the reflective layer may include a flexible film that includes biaxially-oriented polyethylene terephthalate (boPET).

The method 800 can further include connecting the one or more light sources to a controller, as described above, and optionally to a triggering mechanism.

Embodiments of the subject matter and the functional operations described in this specification can be implemented in digital electronic circuitry, in tangibly embodied computer software or firmware, in computer hardware, including the structures disclosed in this specification and their structural equivalents, or in combinations of one or more of them. Embodiments of the subject matter described in this specification can be implemented as one or more computer programs, i.e., one or more modules of computer program instructions encoded on a tangible non transitory program carrier for execution by, or to control the operation of, data processing apparatus. Alternatively or in addition, the program instructions can be encoded on an artificially generated propagated signal, e.g., a machine generated electrical, optical, or electromagnetic signal, that is generated to encode information for transmission to suitable receiver apparatus for execution by a data processing apparatus. The computer storage medium can be a machine-readable storage device, a machine-readable storage substrate, a random or serial access memory device, or a combination of one or more of them. The computer storage medium is not, however, a propagated signal.

The term "data processing apparatus" encompasses all kinds of apparatus, devices, and machines for processing data, including by way of example a programmable processor, a computer, or multiple processors or computers. The apparatus can include special purpose logic circuitry, e.g., an FPGA (field programmable gate array) or an ASIC (application specific integrated circuit). The apparatus can also include, in addition to hardware, code that creates an execution environment for the computer program in question, e.g., code that constitutes processor firmware, a protocol stack, a database management system, an operating system, or a combination of one or more of them.

A computer program (which may also be referred to or described as a program, software, a software application, a module, a software module, a script, or code) can be written in any form of programming language, including compiled or interpreted languages, or declarative or procedural languages, and it can be deployed in any form, including as a standalone program or as a module, component, subroutine, or other unit suitable for use in a computing environment. A computer program may, but need not, correspond to a file in a file system. A program can be stored in a portion of a file that holds other programs or data, e.g., one or more scripts stored in a markup language document, in a single file dedicated to the program in question, or in multiple coordinated files, e.g., files that store one or more modules, sub programs, or portions of code. A computer program can be deployed to be executed on one computer or on multiple computers that are located at one site or distributed across multiple sites and interconnected by a communication network.

As used in this specification, an "engine," or "software engine," refers to a software implemented input/output system that provides an output that is different from the input. An engine can be an encoded block of functionality, such as a library, a platform, a software development kit ("SDK"), or an object. Each engine can be implemented on any appropriate type of computing device, e.g., servers, mobile phones, tablet computers, notebook computers, music players, e book readers, laptop or desktop computers, PDAs, smart phones, or other stationary or portable devices, that includes one or more processors and computer readable media. Additionally, two or more of the engines may be implemented on the same computing device, or on different computing devices.

The processes and logic flows described in this specification can be performed by one or more programmable computers executing one or more computer programs to perform functions by operating on input data and generating output. The processes and logic flows can also be performed by, and apparatus can also be implemented as, special purpose logic circuitry, e.g., an FPGA (field programmable gate array) or an ASIC (application specific integrated circuit).

Computers suitable for the execution of a computer program include, by way of example, can be based on general or special purpose microprocessors or both, or any other kind of central processing unit. Generally, a central processing unit will receive instructions and data from a read only memory or a random access memory or both. The essential elements of a computer are a central processing unit for performing or executing instructions and one or more memory devices for storing instructions and data. Generally, a computer will also include, or be operatively coupled to receive data from or transfer data to, or both, one or more mass storage devices for storing data, e.g., magnetic, magneto optical disks, or optical disks. However, a computer need not have such devices. Moreover, a computer can be embedded in another device, e.g., a mobile telephone, a personal digital assistant (PDA), a mobile audio or video player, a game console, a Global Positioning System (GPS) receiver, or a portable storage device, e.g., a universal serial bus (USB) flash drive, to name just a few.

Computer readable media suitable for storing computer program instructions and data include all forms of non volatile memory, media and memory devices, including by way of example semiconductor memory devices, e.g., EPROM, EEPROM, and flash memory devices; magnetic disks, e.g., internal hard disks or removable disks; magneto optical disks; and CD ROM and DVD ROM disks. The processor and the memory can be supplemented by, or incorporated in, special purpose logic circuitry.

To provide for interaction with a user, embodiments of the subject matter described in this specification can be implemented on a computer having a display device, e.g., a CRT (cathode ray tube) monitor, an LCD (liquid crystal display) monitor, or an OLED display, for displaying information to the user, as well as input devices for providing input to the computer, e.g., a keyboard, a mouse, or a presence sensitive display or other surface. Other kinds of devices can be used to provide for interaction with a user as well; for example, feedback provided to the user can be any form of sensory feedback, e.g., visual feedback, auditory feedback, or tactile feedback; and input from the user can be received in any form, including acoustic, speech, or tactile input. In addition, a computer can interact with a user by sending resources to and receiving resources from a device that is used by the user; for example, by sending web pages to a web browser on a user's client device in response to requests received from the web browser.

Embodiments of the subject matter described in this specification can be implemented in a computing system that includes a back end component, e.g., as a data server, or that includes a middleware component, e.g., an application server, or that includes a front end component, e.g., a client computer having a graphical user interface or a Web browser through which a user can interact with an implementation of the subject matter described in this specification, or any combination of one or more such back end, middleware, or front end components. The components of the system can be interconnected by any form or medium of digital data communication, e.g., a communication network. Examples of communication networks include a local area network ("LAN") and a wide area network ("WAN"), e.g., the Internet.

The computing system can include clients and servers. A client and server are generally remote from each other and typically interact through a communication network. The relationship of client and server arises by virtue of computer programs running on the respective computers and having a client server relationship to each other.

While this specification contains many specific implementation details, these should not be construed as limitations on the scope of any invention or of what may be claimed, but rather as descriptions of features that may be specific to particular embodiments of particular inventions. Certain features that are described in this specification in the context of separate embodiments can also be implemented in combination in a single embodiment. Conversely, various features that are described in the context of a single embodiment can also be implemented in multiple embodiments separately or in any suitable subcombination. Moreover, although features may be described above as acting in certain combinations and even initially claimed as such, one or more features from a claimed combination can in some cases be excised from the combination, and the claimed combination may be directed to a subcombination or variation of a subcombination.

Similarly, while operations are depicted in the drawings in a particular order, this should not be understood as requiring that such operations be performed in the particular order shown or in sequential order, or that all illustrated operations be performed, to achieve desirable results. In certain circumstances, multitasking and parallel processing may be advantageous. Moreover, the separation of various system modules and components in the embodiments described above should not be understood as requiring such separation in all embodiments, and it should be understood that the described program components and systems can generally be integrated together in a single software product or packaged into multiple software products.

Particular embodiments of the subject matter have been described. Other embodiments are within the scope of the following claims. For example, the actions recited in the claims can be performed in a different order and still achieve desirable results. As one example, the processes depicted in the accompanying figures do not necessarily require the particular order shown, or sequential order, to achieve desirable results. In certain implementations, multitasking and parallel processing may be advantageous.

## Claims

1. A self-cleaning device comprising:
a rigid or semi-rigid surface covered with a photocatalyst;
one or more light sources disposed under or above the surface; and
a triggering mechanism that activates a cleaning cycle by activating the one or more light sources.

2. The self-cleaning device of claim 1, wherein the one or more light sources comprise one or more light emitting diodes that emit light within a particular light spectrum that includes a ultraviolet (UV) light spectrum, a visible light spectrum, or a combination of the UV light spectrum and the visible light spectrum.

3. The self-cleaning device of claim 1 or 2, wherein the one or more light sources are arranged facing an underside of the rigid or semi-rigid surface or are arranged facing a direction that extends substantially in parallel to the rigid or semi-rigid surface.

4. The self-cleaning device of claim 1, 2 or 3, wherein the photocatalyst comprises at least one of titanium dioxide or zinc oxide, for example, at least one of titanium dioxide or zinc oxide doped with one or more elements, the one or more elements comprising one or more of: lithium, sodium, magnesium, iron, cobalt, chromium, gold, vanadium, manganese, carbon, boron, iodine, fluorine, sulfur, nitrogen or rare earth elements.

5. The self-cleaning device of any of the preceding claims, wherein the surface comprises one or more holes that allow light emitted by the one or more light sources to illuminate the surface, wherein the device optionally comprises a transparent or translucent layer arranged above the surface comprising one or more holes that allow light emitted by the one or more light sources to illuminate the surface.

6. The self-cleaning device of any of the preceding claims, wherein the surface is transparent or translucent.

7. The self-cleaning device of any of the preceding claims, wherein the triggering mechanism comprises a touch sensor, and wherein the triggering mechanism is configured to activate the cleaning cycle in response to detecting that the surface has been touched.

8. The self-cleaning device of any of the preceding claims, wherein the triggering mechanism comprises a pressure sensor, and wherein the triggering mechanism is configured to activate the cleaning cycle in response to detecting an increase in pressure applied to the surface.

9. The self-cleaning device of any of the preceding claims, wherein the surface comprises one or more regions that each comprise a corresponding light source that is independently activated to clean a corresponding surface of the region.

10. The self-cleaning device of any of the preceding claims, wherein the device is in the form of a handrail or a flat horizontal surface.

11. The self-cleaning device of any of the preceding claims, further comprising one or more buttons for receiving touch input, wherein the surface comprises an outer surface of the one or more buttons.

12. A method for manufacturing a self-cleaning device comprising:
obtaining a rigid or semi-rigid surface;
coating the surface with a photocatalyst that is activated by light within a particular light spectrum that includes a (UV) light spectrum, a visible light spectrum, or a combination of the UV light spectrum and the visible light spectrum; and
embedding one or more light sources under the surface.

13. The method of claim 12, wherein obtaining the surface comprises creating a pattern of holes that extend through the surface.

14. The method of claim 13, further comprising arranging a transparent or translucent refractive layer above the pattern of holes.

15. The method of claim 12, 13 or 14, wherein coating the surface with a photocatalyst comprises abrading the surface or applying either a primer or adhesive to the surface.
